# EUROPEAN PATENT SPECIFICATION

(11) **EP 1 341 521 B1**
(45) Date of publication and mention of the grant of the patent: **07.01.2009**
(21) Application number: 01274072.6
(22) Date of filing: 16.11.2001
(51) Int. Cl.: A61K 9/14

(54) **NANOPARTICULATE COMPOSITIONS COMPRISING A DRUG AND COPOLYMERS OF VINYL PYRROLIDONE AND VINYL ACETATE AS SURFACE STABILIZERS**
NANOPARTIKEL BESTEHEND AUS EINEM ARZNEISTOFF UND COPOLYMEREN VON VINYL PYRROLIDON UND VINYL ACETAT ALS OBERFLÄCHENSTABILISATOREN
COMPOSITIONS NANOPARTICULAIRES COMPRENANT DES COPOLYMERES COMME STABILISATEURS DE SURFACE

(30) Priority: 20.11.2000 US 715117
(43) Date of publication of application: 10.09.2003
(73) Proprietor: Elan Pharma International Limited, Athlone County Westmeath (IE)
(72) Inventor: BOSCH, Henry, William, Bryn Mawr, PA 19010 (US); RYDE, Niels, P., Malvern, PA 19355 (US)
(74) Representative: Ryan, Anne Mary
(86) International application number: PCT/US2001/043111
(87) International publication number: WO 2002/094215

(56) References cited:
- EP-A- 0 818 450
- WO-A-00/18374
- WO-A-02/24163
- WO-A-93/13773
- US-A- 5 118 528
- US-A- 5 595 762
- ZINGONE G ET AL: "Characterization and dissolution study of solid dispersions of theophylline and indomethacin with PVP/VA copolymers" STP PHARMA SCIENCES, PARIS, FR, vol. 2, no. 2, 1992, pages 186-192, XP002111752 ISSN: 1157-1489
- MATSUMOTO T ET AL: "PHYSICAL PROPERTIES OF SOLID MOLECULAR DISPERSIONS OF INDOMETHACIN WITH POLY(VINYLPYRROLIDONE) AND POLY(VINYLPYRROLIDONE-CO-VINYL- ACETATE) IN RELATION TO INDOMETHACIN CRYSTALLIZATION" PHARMACEUTICAL RESEARCH, NEW YORK, NY, US, vol. 16, no. 11, November 1999 (1999-11), pages 1722-1728, XP000987250 ISSN: 0724-8741
- HULSMANN S ET AL: "Melt extrusion - an alternative method for enhancing the dissolution rate of 17beta-estradiol hemihydrate" EUROPEAN JOURNAL OF PHARMACEUTICS AND BIOPHARMACEUTICS, ELSEVIER SCIENCE PUBLISHERS B.V., AMSTERDAM, NL, vol. 49, no. 3, 2 May 2000 (2000-05-02), pages 237-242, XP004257163 ISSN: 0939-6411
- BOGDANOVA, SV. ET AL.: "Solid Dispersions of Isopropylantipyrin" LABO-PHARMA, PROBL. TECH. , vol. 32, no. 348, 1984, pages 835-837, XP001097528
- VOJNOVIC D: "FORMULATION AND EVALUATION OF VINYLPYRROLIDONE/VINYLACETATE COPOLYMER MICROSPHERES WITH GRISEOFULVIN" JOURNAL OF MICROENCAPSULATION, TAYLOR AND FRANCIS INC. LONDON, GB, vol. 10, no. 1, 1993, pages 89-99, XP000334996 ISSN: 0265-2048

## Description

The present invention is directed to nanoparticulate formulations of a drug having at least one copolymer of vinyl pyrrolidone and vinyl acetate adsorbed on the surface of the drug as a surface stabilizer, and methods of making and using such compositions.

Nanoparticulate compositions, first described in U.S. PatentNo. 5,145,684 ("the '684 patent"), are particles consisting of a poorly soluble therapeutic or diagnostic agent having adsorbed onto the surface thereof a non-crosslinked surface stabilizer. The '684 patent describes the use of a variety of surface stabilizers for nanoparticulate compositions. The use of copolymers of vinyl pyrrolidone and vinyl acetate as a surface stabilizer for nanoparticulate compositions, or any other component of such compositions, is not described by the '684 patent.

The '684 patent describes a method of screening drugs to identify useful surface stabilizers that enable the production of a nanoparticulate composition. Not all surface stabilizers will function to produce a stable, non-agglomerated nanoparticulate composition for all drugs. Moreover, known surface stabilizers may be unable to produce a stable, non-agglomerated nonoparticulate composition for certain drugs. Thus, there is a need in the art to identify new surface stabilizers useful in making nanoparticulate compositions. Additionally, such new surface stabilizers may have superior properties over prior known surface stabilizers.

There is a need in the art for new surface stabilizers for nanoparticulate compositions of poorly soluble drugs. In addition, there is a need in the art for surface stabilizers useful in preparing nanoparticulate compositions of drugs, in which prior known surface stabilizers are ineffective. The present invention satisfies these needs.

### SUMMARY OF THE INVENTION

The present invention is directed to nanoparticulate compositions comprising a drug having at least one copolymer of vinyl pyrrolidone and vinyl acetate adsorbed on the surface thereof as a surface stabilizer in an amount sufficient to maintain an effective average particle size of less than 2000 nm, in which the copolymer of vinyl pyrrolidone and vinyl acetate has from 40% up to 99.9% vinyl pyrrolidone and 0.1 % up to 60% vinyl acetate, with the proviso that the composition does not comprise dioctyl sodium sulfosuccinate.

Another aspect of the invention is directed to pharmaceutical compositions comprising a nanoparticulate composition of the invention. The pharmaceutical composition preferably comprises a drug having at least one copolymer of vinyl pyrrolidone and vinyl acetate adsorbed on the surface thereof as a surface stabilizer in an amount sufficient to maintain an effective average particle size of less than 2000 nm, in which the copolymer of vinyl pyrrolidone and vinyl acetate has from 40% up to 99.9% vinyl pyrrolidone and 0.1% up to 60% vinyl acetate, and a pharmaceutically acceptable carrier, as well as any desired excipients, with the proviso that the composition does not comprise dioctyl sodium sulfosuccinate.

This invention further discloses a method of making a nanoparticulate composition comprising an organic drug having at least one copolymer of vinyl pyrrolidone and vinyl acetate as a surface stabilizer adsorbed on the surface thereof in which the effective average particle size is less than 2000 nm. Such a method comprises contacting said drug with at least one copolymer of vinyl pyrrolidone and vinyl acetate, in which the copolymer of vinyl pyrrolidone and vinyl acetate has from 40% up to 99.9% vinyl pyrrolidone and 0.1% up to 60% vinyl acetate, for a time and under conditions sufficient to provide a nanoparticle/copolymer composition, with the proviso that the composition does not comprise dioctyl sodium sulfosuccinate. The copolymer surface stabilizers can be contacted with the drug either before, during, or after size reduction of the drug.

The present invention is further directed to use of a therapeutically effective amount of a nanoparticulate drug/copolymer composition according to the invention as a medicament.

Both the foregoing general description and the following detailed description are exemplary and explanatory and are intended to provide further explanation of the invention as claimed. Other objects, advantages, and novel features will be readily apparent to those skilled in the art from the following detailed description of the invention.

The present invention is directed to a composition comprising nanoparticulate drug having at least one copolymer of vinyl pyrrolidone and vinyl acetate as a surface stabilizer adsorbed on the surface thereof, and methods of making and using such nanoparticulate compositions.

### A. Compositions

The compositions of the invention comprise nanoparticulate drug and at least one copolymer of vinyl pyrrolidone and vinyl acetate as a surface stabilizer adsorbed to the surface of the drug. Surface stabilizers useful herein physically adhere to 1the surface of the nanoparticulate drug, but do not chemically react with the drug or itself. Individually adsorbed molecules of the surface stabilizer are essentially free of intermolecular cross-linkage.

The present invention also includes nanoparticulate compositions having at least one copolymer of vinyl pyrrolidone and vinyl acetate as a surface stabilizer adsorbed on the surface thereof, formulated into compositions together with one or more non-toxic physiologically acceptable carriers, adjuvants, or vehicles, collectively referred to as carriers. The compositions can be formulated for parenteral injection, oral administration in solid or liquid form, rectal or topical administration,

### 1. Drug Particles

The nanoparticles of the invention comprises a therapeutic or diagnostic agent, collectively referred to as a "drug" A therapeutic agent can be a pharmaceutical agent, including biologics such as proteins, peptides, and nucleotides, or a diagnostic agent, such as a contrast agent, including x-ray contrast agents. The drug exists either as a discrete, crystalline phase, or as an amorphous phase. The crystalline phase differs from a non-crystalline or amorphous phase which results from precipitation techniques, such as those described in EP Patent No. 275,796.

The invention can be practiced with a variety of drugs. The drug is preferably present in an essentially pure form, is poorly soluble, and is dispersible in at least one liquid medium. By "poorly soluble" it is meant that the drug has a solubility in the liquid dispersion medium of less than about 10 mg/mL, and preferably of less than about 1 mg/mL.

The drug can be selected from a variety of known classes of drugs, including, for example, proteins, peptides, nucleotides, anti-obesity drugs, nutriceuticals, corticosteroids, elastase inhibitors, analgesics, anti-fungals, oncology therapies, anti-emetics, analgesics, cardiovascular agents, anti-inflammatory agents, anthelmintics, anti-arrhythmic agents, antibiotics (including penicillins), anticoagulants, antidepressants, antidiabetic agents, antiepileptics, antihistamines, antihypertensive agents, andmuscarinic agents, antimycobacterial agents, antineoplastic agents, immunosuppressants, antithyroid agents, antiviral agents, anxiolytic sedatives (hypnotics and neuroleptics), astringents, beta-adrenoceptor blocking agents, blood products and substitutes, cardiac inotropic agents, contrast media, corticosteroids, cough suppressants (expectorants and mucolytics), diagnostic agents, diagnostic imaging agents, diuretics, dopaminergics (antiparkinsonian agents), haemostatics, immunogical agents, lipid regulating agents, muscle relaxants, parasympathomimetics, parathyroid calcitonin and biphosphonates, prostaglandins, radiopharmaceuticals, sex hormones (including steroids), anti-allergic agents, stimulants and anoretics, sympathomimetics, thyroid agents, vasodilators and xanthines.

The drugs are commercially available and/or can be prepared by techniques known in the art.

### 2. Copolymer Surface Stabilizers

Commercially available copolymers of vinyl pyrrolidone and vinyl acetate are Plasdone^{®} S630 (ISP) and Kollidon**^{®}** VA 64 (BASF), which contain vinyl pyrrolidone and vinyl acetate in a 60:40 ratio. Other copolymers of vinyl pyrrolidone and vinyl acetate can also be used in the invention. The copolymer contains at least 40% vinyl pyrrolidone. Other useful copolymers contain vinyl pyrrolidone and vinyl acetate in ratios of, for example, 90:10,80:20,70:30, and 50:50. The amount of vinyl pyrrolidone can range from about 40% up to about 99.9%, and the amount of vinyl acetate can range from about 0.1% up to about 60%.

Two or more surface stabilizers can be used in combination.

### 3. Auxiliary Surface Stabilizers

The compositions of the invention can also include one or more auxiliary surface stabilizers in addition to the at least one copolymer of vinyl pyrrolidone and vinyl acetate. Suitable auxiliary surface stabilizers can preferably be selected from known organic and inorganic pharmaceutical excipients. Such excipients include various polymers, low molecular weight oligomers, natural products, and surfactants. Preferred surface stabilizers include nonionic and ionic surfactants. Two or more surface auxiliary stabilizers can be used in combination.

Representative examples of auxiliary surface stabilizers include cetyl pyridinium chloride, gelatin, casein, lecithin (phosphatides), dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g.*, the commercially available Tweens^{®} such as *e.g*., Tween 20^{®} and Tween 80^{®} (ICI Specialty Chemicals)); polyethylene glycols (*e.g*., Carbowaxes 3350^{®} and 1450^{®}, and Carbopol 934^{®} (Union Carbide)), dodecyl trimethyl ammonium bromide, polyoxyethylene stearates; colloidal silicon dioxide, phosphates, sodium dodecylsulfate, carboxymethylcellulose calcium, hydroxypropyl celluloses (*e.g*., HPC, HPC-SL, and HPC-L), hydroxypropyl methylcellulose (HPMC), carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol (PVA), polyvinylpyrrolidone (PVP), 4-(1,1,3,3-tetramethylbutyl)-phenol polymer with ethylene oxide and formaldehyde (also known as tyloxapol, superione, and triton), poloxamers (*e.g*., Pluronics F68^{®} and F108^{®}, which are block copolymers of ethylene oxide and propylene oxide); poloxamines (*e.g*., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); a charged phospholipid such as dimyristoyl phophatidyl glycerol, dioctylsulfosuccinate (DOSS); Tetronic 1508^{®} (T-1508) (BASF Wyandotte Corporation ), dialkylesters of sodium sulfosuccinic acid (*e.g*., Aerosol OT^{®}, which is a dioctyl ester of sodium sulfosuccinic acid (Cytec Industries, West Paterson, NJ)); Duponol P^{®}, which is a sodium lauryl sulfate (DuPont); Triton X-200^{®}, which is an alkyl aryl polyether sulfonate (Union Carbide); Crodestas F-110^{®}, which is a mixture of sucrose stearate and sucrose distearate (Croda Inc.); p-isononylphenoxypoly-(glycidol), also known as Olin-IOG^{®} or Surfactant 10-G^{®} (Olin Chemicals, Stamford, CT); Crodestas SL-40^{®} (Croda, Inc.); decanoyl-N methylglucamide; n-decyl β-D-glucapyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside:and octyl β-D-thioglucopyranoside.

Most of these surface stabilizers are known pharmaceutical excipients and are described in detail in the *Handbook of Pharmaceutical Excipients,* published jointly by the American Pharmaceutical Association and The Pharmaceutical Society of Great Britain (The Pharmaceutical Press, 2000), specifically incorporated by reference. The surface stabilizers are commercially available and/or can be prepared by techniques known in the art.

### 4. Nanoparticulate Drug/Copolymer Particle Size

The compositions of the invention contain nanoparticles which have an effective average particle size of less than about 2000 nm (i.e., 2 microns), more preferably less than about 1500 nm, less than about 1000 nm, less than about 800 nm, less than about 600 nm, less than about 400 nm, less than about 300 nm, less than about 250 nm, less than about 100 nm, or less than about 50 nm, as measured by light-scattering methods, microscopy, or other appropriate methods. By "an effective average particle size of less than about 2000 nm" it is meant that at least 50% of the drug particles have a particle size of less than about 2000 nm when measured by light scattering techniques. Preferably, at least 70% of the drug particles have a particle size of less than about 2000 nm, more preferably at least 90% of the drug particles have a particle size of less than about 2000 nm, and even more preferably at least about 95% of the particles have a particle size of less than about 2000 nm.

### 5. Concentration of Nanoparticulate Drug and Stabilizer

The relative amount of drug and one or more surface stabilizers can vary widely. The optimal amount of the surface stabilizers can depend, for example, upon the particular active agent selected, the hydrophilic lipophilic balance (HLB), melting point, and water solubility of the copolymer, and the surface tension of water solutions of the stabilizer, etc.

The concentration of the one or more surface stabilizers can vary from about 0.01 to about 90%, from about 1 to about 75%, from about 10 to about 60%, and from about 10 to about 55% by weight based on the total combined weight of the drug substance and surface stabilizer, not including other excipients.

The concentration of the drug can vary from about 99.9% to about 0.1%, from about 80% to about 5.0%, or from about 50% to about 10%, by weight based on the total combined weight of the drug substance and surface stabilizer, not including other excipients.

### B. Methods of Making Nanoparticulate Formulations

The nanoparticulate drug compositions can be made using, for example, milling or precipitation techniques. Exemplary methods of making nanoparticulate compositions are described in the '684 patent. Methods of making nanoparticulate compositions are also described in U.S. Patent. No. 5,518,187, for "Method of Grinding Pharmaceutical Substances;" U.S. Patent No. 5,718,388, for "Continuous Method of Grinding Pharmaceutical Substances;" U.S. Patent No. 5,862,999, for "Method of Grinding Pharmaceutical Substances;" U.S. Patent No. 5,665,331, for "Co-Microprecipitation of Nanoparticulate Pharmaceutical Agents with Crystal Growth Modifiers;" U.S. Patent No. 5,662,883, for "Co-Microprecipitation of Nanoparticulate Pharmaceutical Agents with Crystal Growth Modifiers;" U.S. Patent No. 5,560,932, for "Microprecipitation of Nanoparticulate Pharmaceutical Agents;" U.S. Patent No. 5,543,133, for "Process of Preparing X-Ray Contrast Compositions Containing Nanoparticles;" U.S. Patent No. 5,534,270, for "Method of Preparing Stable Drug Nanoparticles;" U.S. Patent No. 5,510,118, for "Process of Preparing Therapeutic Compositions Containing Nanoparticles;" and U.S. Patent No. 5,470,583, for "Method of Preparing Nanoparticle Compositions Containing Charged Phospholipids to Reduce Aggregation".

### 1. Milling to obtain Nanoparticulate Drug Dispersions

Milling of aqueous drug to obtain a nanoparticulate dispersion comprises dispersing drug particles in a liquid dispersion medium in which the drug is poorly soluble, followed by applying mechanical means in the presence of grinding media to reduce the particle size of the drug to the desired effective average particle size. The dispersion medium can be, for example, water, safflower oil, ethanol, t-butanol, glycerin, polyethylene glycol (PEG), hexane, or glycol.

The particles can be reduced in size in the presence of at least one copolymer of vinyl pyrrolidone and vinyl acetate surface stabilizer. Alternatively, the particles can be contacted with one or more surface stabilizers after attrition. Other compounds, such as a diluent, can be added to the drug/surface stabilizer composition during the size reduction process. Dispersions can be manufactured continuously or in a batch mode. The resultant nanoparticulate drug dispersion can be utilized in solid or liquid dosage formulations.

### 2. Precipitation to Obtain Nanoparticulate Drug Compositions

Another method of forming the desired nanoparticulate composition is by microprecipitation. This is a method of preparing stable dispersions of poorly soluble drugs in the presence of one or more surface stabilizers and one or more colloid stability enhancing surface active agents free of any trace toxic solvents or solubilized heavy metal impurities. Such a method comprises, for example: (1) dissolving the poorly soluble drug in a suitable solvent; (2) adding the formulation from step (1) to a solution comprising at least one copolymer surface stabilizer to form a clear solution; and (3) precipitating the formulation from step (2) using an appropriate non-solvent. The method can be followed by removal of any formed salt, if present, by dialysis or diafiltration and concentration of the dispersion by conventional means. The resultant nanoparticulate drug dispersion can be utilized in solid or liquid dosage formulations.

### C. Methods of Using Nanoparticulate Drug Formulations Comprising One or More Surface Stabilizers

The nanoparticulate compositions of the present invention can be administered to humans and animals via any conventional means, including orally, rectally, parenterally (intravenous, intramuscular, or subcutaneous), intracisternally, pulmonary, intravaginally, intraperitoneally, locally (powders, ointments or drops), or as a buccal or nasal spray.

Compositions suitable for parenteral injection may comprise physiologically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents, or vehicles including water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate compositions may also contain adjuvants such as preserving, wetting, emulsifying, and dispensing agents. Prevention of the growth of microorganisms can be ensured by various antibacterial and antifungal agents, such as parabens, chlorobutanol, phenol, and sorbic acid. It may also be desirable to include isotonic agents, such as sugars and sodium chloride. Prolonged absorption of the injectable pharmaceutical form can be brought about by the use of agents delaying absorption, such as aluminum monostearate and gelatin.

Solid dosage forms for oral administration include capsules, tablets, pills, powders, and granules. In such solid dosage forms, the active compound is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to the active compounds, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances.

Besides such inert diluents, the composition can also include adjuvants, such as wetting agents, emulsifying and suspending agents, sweetening, flavoring, and perfuming agents.

Actual dosage levels of active ingredients in the nanoparticulate compositions of the invention may be varied to obtain an amount of active ingredient that is effective to obtain a desired therapeutic response for a particular composition and method of administration. The selected dosage level therefore depends upon the desired therapeutic effect, the route of administration, the potency of the administered drug the desired duration of treatment, and other factors.

The total daily dose of the compounds of this invention administered to a host in single or divided dose may be in amounts of, for example, from about 1 nanomol to about 50 micromoles per kilogram of body weight. Dosage unit compositions may contain such amounts of such submultiples thereof as may be used to make up the daily dose. It will be understood, however, that the specific dose level for any particular patient will depend upon a variety of factors including the body weigh, general health, sex, diet, time and route of administration, potency of the administered drug, rates of absorption and excretion, combination with other drugs and the severity of the particular disease being treated.

The following examples are given to illustrate the present invention.

### Example 1

The purpose of this example was to prepare a nanoparticulate dispersion of a naproxen composition comprising a copolymer of vinyl pyrrolidone and vinyl acetate. Naproxen is a non-steroidal anti-inflammatory drug (NSAID) used in the treatment of rheumatoid conditions.

5% (w/w) of naproxen and 1% Plasdone**^{®}** S-630 (60% vinyl pyrrolidone, 40% vinyl acetate) (ISP Technologies, Inc.) was milled using a Dyno^{®} Mill (Type: KDL; Mfg.: Willy A Bachofen AG, Basel Switzerland) equipped with a 150 cc batch chamber using a 500 µm milling media (PolyMill^{®} 500; Dow Chemical) for 2 hrs at ca. 10°C

Following milling, the nanoparticulate naproxen dispersion had a mean particle size of 96 nm, with 90% of the particles having a size of less than 141 nm.

### Example 2

The purpose of this example was to prepare a nanoparticulate dispersion of a nifedipine composition comprising a copolymer of vinyl pyrrolidone and vinyl acetate. Nifedipine is a poorly water-soluble calcium channel blocking agent. The drug affects the movement of calcium into heart and blood vessel cells, and causes a relaxing effect of the muscles to allow an increased amount of blood flow into the heart. Nifedipine is useful in treating angina pectoris (chest pain), and to help reduce blood pressure (antihypertensive).

An aqueous solution of 1% Plasdone® S-630 (60% vinyl pyrrolidone and 40% vinyl acetate) (ISP Technologies, Inc.) and 0.05% sodium lauryl sulfate (SLS) (Spectrum) was prepared by dissolving 0.85 g of polymer and 4.59 g of a 1% SLS solution in 75.66 g of deionized water. The stabilizer solution was mixed with 4.25 g of nifedipine (5% w/w) and charged into the chamber of a DYNO^{®}-Mill Type KDL media mill (Willy Bachofen AG, Basel, Switzerland) along with 500 micron polymeric media (PolyMill^{®} 500; Dow Chemical). The mill was operated for 2 hours and yielded a stable colloidal dispersion of drug substance having a mean particle size of 132 nm, with 90% of the particles having a size of less than 193 nm:

### Example 3

The purpose of this example was to prepare a nanoparticulate dispersion of a ketoprofen composition comprising a copolymer of vinyl pyrrolidone and vinyl acetate. Ketoprofen is a nonsteroidal anti-inflammatory drug (NSAID) effective in treating fever, pain, and inflammation in the body.

An aqueous solution of 1% Plasdone® S-630 (60% vinyl pyrrolidone and 40% vinyl acetate) (ISP Technologies, Inc.) and 0.05% sodium lauryl sulfate (SLS) (Spectrum) was prepared by dissolving 0.85 g of polymer and 4.26 g of a 1% SLS solution in 75.71 g of deionized water. The stabilizer solution was mixed with 4.25 g of ketoprofen (Wyckoff; 5% w/w) and charged into the chamber of a DYNO^{®}-Mill Type KDL media mill (Willy Bachofen AG, Basel, Switzerland) along with 500 micron polymeric media (PolyMill^{®} 500; Dow Chemical). The mill was operated for 1 hour and yielded a stable colloidal dispersion of drug substance having a mean particle size of 256 nm, with 90% of the particles having a size of less than 355 nm.

### Example 4

The purpose of this example was to prepare a nanoparticulate dispersion of a triamcinolone acetonide composition comprising a copolymer of vinyl pyrrolidone and vinyl acetate. Triamcinolone acetonide is a corticosteroid used as an antiallergic agent.

An aqueous solution of 1% Plasdone® S-630 (60% vinyl pyrrolidone and 40% vinyl acetate) (ISP Technologies, Inc:) and 0.05% sodium lauryl sulfate (SLS) (Spectrum) was prepared by dissolving 0.85 g of polymer and 4.30 g of a 1% SLS solution in 76.10 g of deionized water. The stabilizer solution was mixed with 4.26 g of triamcinolone acetonide (5% w/w) and charged into the chamber of a DYNO^{®}-Mill Type KDL media mill (Willy Bachofen AG, Basel, Switzerland) along with 500 micron polymeric media (PolyMill^{®} 500; Dow Chemical). The mill was operated for 2 hours and yielded a colloidal dispersion of drug substance having a mean particle size of 121 nm, with 90% of the particles-having a size of less than 194 nm.

### Example 5

The purpose of this example was to prepare a nanoparticulate dispersion of a nanoparticulate diagnostic imaging agent, benzoic acid, 3,5-bis(acetylamino) 2,4,6-triodo, 4-(ethyl-3-ethoxy-2-butenoate) ester (WIN 68209) composition comprising a copolymer of vinyl pyrrolidone and vinyl acetate.

An aqueous solution of 1% of a copolymer of 80% vinyl pyrrolidone and 20% vinyl acetate (Polysciences Inc., Warrington, PA) was prepared by dissolving 0.85 g of polymer in 79.91 g of deionized water. The stabilizer solution was mixed with 4.26 g of WIN 68209 (5% drug) and charged into the chamber of a DYNO^{®}-Mill Type KDL media mill (Willy Bachofen AG, Basel, Switzerland) along with 500 micron polymeric media (PolyMill 500; Dow Chemical). The mill was operated for 1 hour and yielded a stable colloidal dispersion of WIN 68209 having a mean particle size of 242 nm, with 90% of the particles having a size of less than 347 nm.

### Example 6

The purpose of this example was to prepare a nanoparticulate dispersion of a nanoparticulate diagnostic imaging agent, WIN 68209, composition comprising a copolymer of vinyl pyrrolidone and vinyl acetate.

An aqueous solution of 1%,of a copolymer of 80% vinyl pyrrolidone and 20% vinyl acetate (Polysciences Inc., Warrington, PA) and 1% SLS was prepared by dissolving 0.85 g of polymer and 4.25 g of a 1% SLS solution in 75.67 g of deionized water. The stabilizer solution was mixed with 4.26 g of WIN 68209 (5% drug) and charged into the chamber of a DYNO^{®} Mill Type KDL media mill (Willy Bachofen AG, Basel, Switzerland) along with 500 micron polymeric media (PolyMill 500; Dow Chemical). The mill was operated for 1 hour and yielded a stable colloidal dispersion of WIN 68209 having a mean particle size of 188 nm, with 90% of the particles having a size of less than 308 nm.

### Example 7

The purpose of this example was to prepare a nanoparticulate dispersion of a nanoparticulate diagnostic imaging agent, WIN 68209, composition comprising a copolymer of vinyl pyrrolidone and vinyl acetate.

An aqueous solution of 1% of a copolymer of 50% vinyl pyrrolidone and 50% vinyl acetate (Polysciences Inc., Warrington, PA) and 0.05% SLS (Spectrum) was prepared by dissolving 0.85 g of polymer and 0.043 g of SLS in 79.86 g of deionized water. The stabilizer solution was mixed with 4.25 g of WIN 68209 (5% drug) and charged into the chamber of a DYNO^{®}-Mill Type KDL media mill (Willy Bachofen AG, Basel, Switzerland) along with 500 micron polymeric media (PolyMill 500; Dow Chemical). The mill was operated for 2 hours and yielded a stable colloidal dispersion of WIN 68209 having a mean particle size of 96 nm, with 90% of the particles having a size of less than 143 nm.

## Claims

1. A nanoparticulate composition comprising a drug having at least one copolymer of vinyl pyrrolidone and vinyl acetate adsorbed on the surface thereof as a surface stabilizer in an amount sufficient to maintain an effective average particle size of less than 2000 nm, in which the copolymer of vinyl pyrrolidone and vinyl acetate has from 40% up to 99.9% vinyl pyrrolidone and 0.1% up to 60% vinyl acetate, with the proviso that the composition does not comprise dioctyl sodium sulfosuccinate.

2. A method of making a nanoparticulate composition comprising an organic drug having at least one copolymer of vinyl pyrrolidone and vinyl acetate as a surface stabilizer adsorbed on the surface thereof in which the effective average particle size is less than 2000 nm, said method comprising contacting said drug with at least one copolymer of vinyl pymolidone and vinyl acetate, in which the copolymer of vinyl pyrrolidone and vinyl acetate has from 40% up to 99.9% vinyl pyrrolidone and 0.1 % up to 60% vinyl acetate, with the proviso that the composition does not comprise dioctyl sodium sulfosuccinate.

3. A method according to Claim 2, wherein the contacting step comprises:
(a) dissolving the drug in a solvent;
(b) adding the solubilized drug to a solution comprising at least one copolymer of vinyl pyrrolidone and vinyl acetate to form a clear solution; and
(c) precipitating the solubilized drug having a copolymer of vinyl pyrrolidone and vinyl acetate as a surface stabilizer using a non-solvent.

4. A composition according to Claim 1 or a method according to Claim 2 or 3, in which the copolymer of vinyl pyrrolidone and vinyl acetate has from 50% up to 99.9% vinyl pyrrolidone and 0.1% up to 50% vinyl acetate.

5. A composition according to Claim 1 or 4 or a method according to any one of Claims 2 to 4, in which the copolymer of vinyl pyrrolidone and vinyl acetate has 60% vinyl pyrrolidone and 40% vinyl acetate.

6. A composition according to any one of Claims 1, 4 and 5 or a or a method according to any one of Claims 2 to 5, in which the copolymer of vinyl pyrrolidone and vinyl acetate has 80% vinyl pyrrolidone and 20% vinyl acetate.

7. A composition according to any one of Claims 1 and 4 to 6, or a method according to any one of Claims 2 to 6, in which the drug is present in an amount selected from from 99.9% to 0.1%; from 80% to 5.0% and from 50% to 10%, by weight based on the total combined weight of the drug substance and surface stabilizer, not including other excipients.

8. A composition according to any one of Claims 1 and 4 to 7, or a method according to any one of Claims 2 to 7, in which at least one copolymer of vinyl pyrrolidone and vinyl acetate is present in an amount selected from 0.1 to 90%, from 1 to 75%, from 10 to 60% and from 10 to 55%, by weight based on the total combined weight of the drug substance and surface stabilizer, not including other excipients.

9. A composition according to any one of Claims 1 and 4 to 8 or a methods according to any one of Claims 2 to 8, in which the drug is selected from the group consisting of a crystalline phase drug and an amorphous phase drug.

10. A composition according to any one of Claims 1 and 4 to 9 or a method according to any one of Claims 2 to 9, further comprising at least one auxiliary surface stabilizer in addition to the copolymer of vinyl pyrrolidone and vinyl acetate.

11. A composition according to Claim 10 when dependent on any one of Claims 1 and 4 to 9, or a method according to Claim 10 when dependent on any one of Claims 2 to 9, further comprising at least one auxiliary surface stabilizer in addition to at least one copolymer of vinyl pyrrolidone and vinyl acetate.

12. A composition or method according to Claim 11, wherein the auxiliary surface stabilizer comprises lauryl sulphate.

13. A composition according to any one of Claims 1 and 4 to 12 or a method according to any one of Claims 2-12, in which the effective average particle size of the nanoparticle is less than 1500nm.

14. A composition according to any one of Claims 1 and 4 to 13 or a method according to any one of Claims 2-13, in which the effective average particle size of the nanoparticle is less than 1000nm.

15. A composition according to any one of Claims 1 and 4 to 14 or a method according to any one of Claims 2-14, in which the effective average particle size of the nanoparticle is less than 900nm.

16. A composition according to any one of Claims 1 and 4 to 15 or a method according to any one of Claims 2-15, in which the effective average particle size of the nanoparticle is less than 600nm.

17. A composition according to any one of Claims 1 and 4 to 16 or a method according to any one of Claims 2-16, in which the effective average particle size of the nanoparticle is less than 400nm.

18. A composition according to any one of Claims 1 and 4 to 17 or a method according to any one of Claims 2-17, in which the effective average particle size of the nanoparticle is less than 300nm.

19. A composition according to any one of Claims 1 and 4 to 18 or a method according to any one of Claims 2-18, in which the effective average particle size of the nanoparticle is less than 200nm.

20. A composition according to any one of Claims 1 and 4 to 19 or a method according to any one of Claims 2-19, in which the effective average particle size of the nanoparticle is less than 100nm.

21. A composition according to any one of Claims 1 and 4 to 20 or a method according to any one of Claims 2-20, in which the effective average particle size of the nanoparticle is less than 50nm.

22. A composition according to Claim 1 or any one of Claims 4 to 21 as dependent thereon, for use as a medicament.

23. A composition according to Claim 21 for use as a medicament wherein the drug is an organic drug.

24. A pharmaceutical composition comprising a nanoparticulate composition according to Claim 1 or any one of Claims 4 to 21 and a pharmaceutically acceptable carrier.

## Patentansprüche

1. Eine nanopartikuläre Zusammensetzung umfassend ein Arzneimittel, das mindestens ein Copolymer aus Vinylpyrrolidon und Vinylacetat an dessen Oberfläche als Oberflächenstabilisator adsorbiert hat, in einer Menge die ausreicht eine effektive durchschnittliche Partikelgröße von weniger als 2000 nm zu erhalten, wobei das Copolymer aus Vinylpyrrolidon und Vinylacetat von 40% bis zu 99,9% Vinylpyrrolidon und 0,1% bis zu 60% Vinylacetat aufweist, mit der Maßgabe, dass die Zusammensetzung nicht Natriumdioctylsulfosuccinat umfaßt.

2. Ein Verfahren zur Herstellung einer nanopartikulären Zusammensetzung umfassend ein organisches Arzneimittel, das mindestens ein Copolymer aus Vinylpyrrolidon und Vinylacetat als Oberflächenstabilisator an dessen Oberfläche adsorbiert hat, wobei die effektive durchschnittliche Partikelgröße weniger als 2000 nm ist, das genannte Verfahren umfaßt das in Kontakt bringen von genanntem Arzneimittel mit mindestens einem Copolymer aus Vinylpyrrolidon und Vinylacetat, wobei das Copolymer aus Vinylpyrrolidon und Vinylacetat von 40% bis zu 99,9% Vinylpyrrolidon und 0,1 % bis zu 60% Vinylacetat aufweist, mit der Maßgabe, dass die Zusammensetzung nicht Natriumdioctylsulfosuccinat umfaßt.

3. Das Verfahren gemäß Anspruch 2, wobei der Schritt des in Kontakt bringens umfaßt:
(a) Auflösen des Arzneimittels in einem Losungsmittel;
(b) Zugabe des gelösten Arzneimittels zu einer Lösung, die mindestens ein Copolymer aus Vinylpyrrolidon und Vinylacetat umfaßt um eine klare Lösung zu bilden; und
(c) Präzipitieren des gelösten Arzneimittels, das als Oberflächenstabilisator ein Copolymer aus Vinylpyrrolidon und Vinylacetat hat, unter Verwendung eines Nicht-Lösungsmittels.

4. Die Zusammensetzung gemäß Anspruch 1 oder das Verfahren gemäß Anspruch 2 oder 3, wobei das Copolymer aus Vinylpyrrolidon und Vinylacetat von 50% bis zu 99,9% Vinylpyrrolidon und 0,1 % bis zu 50% Vinylacetat aufweist.

5. Die Zusammensetzung gemäß Anspruch 1 oder 4 oder das Verfahren gemäß einem der Ansprüche 2 bis 4, wobei das Copolymer aus Vinylpyrrolidon und Vinylacetat 60% Vinylpyrrolidon und 40% Vinylacetat aufweist.

6. Die Zusammensetzung gemäß einem der Ansprüche 1, 4 und 5 oder das Verfahren gemäß einem der Ansprüche 2 bis 5, wobei das Copolymer aus Vinylpyrrolidon und Vinylacetat 80% Vinylpyrrolidon und 20% Vinylacetat aufweist.

7. Die Zusammensetzung gemäß einem der Ansprüche 1 und 4 bis 6 oder das Verfahren gemäß einem der Ansprüche 2 bis 6, wobei das Arzneimittel in einer Menge ausgewählt aus von 99,9% bis 0,1%, von 80% bis 5% und von 50% bis 10% Gewichtsprozent vorliegt, basierend auf dem totalen kombinierten Gewicht von der Arzneimittelsubstanz und dem Oberflächenstabilisator ohne andere Hilfsstoffe.

8. Die Zusammensetzung gemäß einem der Ansprüche 1 und 4 bis 7 oder das Verfahren gemäß einem der Ansprüche 2 bis 7, wobei mindestens ein Copolymer aus Vinylpyrrolidon und Vinylacetat in in einer Menge ausgewählt aus von 0,1 bis 90%, von 1 bis 75%, von 10 bis 60% und von 10 bis 55% Gewichtsprozent vorliegt, basierend auf dem totalen kombinierten Gewicht von der Arzneimittelsubstanz und dem Oberflächenstabilisator ohne andere Hilfsstoffe.

9. Die Zusammensetzung gemäß einem der Ansprüche 1 und 4 bis 8 oder das Verfahren gemäß einem der Ansprüche 2 bis 8, wobei das Arzneimittel aus der Gruppe ausgewählt ist, bestehend aus einem Arzneimittel in der kristallinen Phase und einem Arzneimittel in der amorphen Phase.

10. Die Zusammensetzung gemäß einem der Ansprüche 1 und 4 bis 9 oder das Verfahren gemäß einem der Ansprüche 2 bis 9, außerdem umfassend mindestens einen Hilfsoberflächenstabilisator, zusätzlich zu dem Copolymer aus Vinylpyrrolidon und Vinylacetat.

11. Die Zusammensetzung gemäß Anspruch 10, wenn dieser abhängig von einem der Ansprüche 1 und 4 bis 9 ist oder das Verfahren gemäß Anspruch 10, wenn dieser abhängig von einem der Ansprüche 2 bis 9 ist, außerdem umfassend mindestens einen Hilfsoberflächenstabilisator, zusätzlich zu dem mindestens einen Copolymer aus Vinylpyrrolidon und Vinylacetat.

12. Die Zusammensetzung oder das Verfahren gemäß Anspruch 11, worin der Hilfsoberflächenstabilisator Laurylsulphat umfaßt.

13. Die Zusammensetzung gemäß einem der Ansprüche 1 und 4 bis 12 oder das Verfahren gemäß einem der Ansprüche 2-12, wobei die effektive durchschnittliche Partikelgröße des Nanopartikels weniger als 1500 nm beträgt.

14. Die Zusammensetzung gemäß einem der Ansprüche 1 und 4 bis 13 oder das Verfahren gemäß einem der Ansprüche 2-13, wobei die effektive durchschnittliche Partikelgröße des Nanopartikels weniger als 1000 nm beträgt.

15. Die Zusammensetzung gemäß einem der Ansprüche 1 und 4 bis 14 oder das Verfahren gemäß einem der Ansprüche 2-14, wobei die effektive durchschnittliche Partikelgröße des Nanopartikels weniger als 800 nm beträgt.

16. Die Zusammensetzung gemäß einem der Ansprüche 1 und 4 bis 15 oder das Verfahren gemäß einem der Ansprüche 2-15, wobei die effektive durchschnittliche Partikelgröße des Nanopartikels weniger als 600 nm beträgt.

17. Die Zusammensetzung gemäß einem der Ansprüche 1 und 4 bis 16 oder das Verfahren gemäß einem der Ansprüche 2-16, wobei die effektive durchschnittliche Partikelgröße des Nanopartikels weniger als 400 nm beträgt.

18. Die Zusammensetzung gemäß einem der Ansprüche 1 und 4 bis 17 oder das Verfahren gemäß einem der Ansprüche 2-17, wobei die effektive durchschnittliche Partikelgröße des Nanopartikels weniger als 300 nm beträgt.

19. Die Zusammensetzung gemäß einem der Ansprüche 1 und 4 bis 18 oder das Verfahren gemäß einem der Ansprüche 2-18, wobei die effektive durchschnittliche Partikelgröße des Nanopartikels weniger als 200 nm beträgt.

20. Die Zusammensetzung gemäß einem der Ansprüche 1 und 4 bis 19 oder das Verfahren gemäß einem der Anspruche 2-19, wobei die effektive durchschnittliche Partikelgröße des Nanopartikels weniger als 100 nm beträgt.

21. Die Zusammensetzung gemäß einem der Ansprüche 1 und 4 bis 20 oder das Verfahren gemäß einem der Ansprüche 2-20, wobei die effektive durchschnittliche Partikelgröße des Nanopartikels weniger als 50 nm beträgt.

22. Die Zusammensetzung gemäß Anspruch 1 oder einem der davon abhängigen Ansprüche 4 bis 21, für die Verwendung als ein Medikament.

23. Die Zusammensetzung gemäß Anspruch 21, für die Verwendung als ein Medikament, wobei das Arzneimittel ein organisches Arzneimittel ist.

24. Eine pharmazeutische Zusammensetzung umfassend die nanoparticuläre Zusammensetzung gemäß Anspruch 1 oder einem der Ansprüche 4 bis 21 und einen pharmazeutisch Hilfsstoff.

## Revendications

1. Composition nanoparticulaire comprenant un médicament comportant au moins un copolymère de vinylpyrrolidone et d'acétate de vinyle adsorbé à sa surface en tant que stabilisateur de surface dans une quantité suffisante pour maintenir une dimension moyenne efficace de particules inférieure à 2000 nm, dans laquelle le copolymère de vinylpyrrolidone et d'acétate de vinyle comporte de 40% jusqu'à 99,9% de vinylpyrrolidone et de 0,1% jusqu'à 60% d'acétate de vinyle, à condition que la composition ne comporte pas de sulfosuccinate de dioctylsodium.

2. Procédé de fabrication d'une composition nanoparticulaire comportant un médicament organique ayant, adsorbé à sa surface, au moins un copolymère de vinylpyrrolidone et d'acétate de vinyle en tant qu'agent stabilisateur de surface, dans lequel la dimension moyenne efficace des particules est inférieure à 2000 nm, ledit procédé consistant à mettre en contact ledit médicament avec au moins un copolymère de vinylpyrrolidone et d'acétate de vinyle, dans lequel le copolymère de vinylpyrrolidone et d'acétate de vinyle contient de 40% à 99,9% de vinylpyrrolidone et de 0,1% à 60% d'acétate de vinyle, à condition que la composition ne comporte pas de sulfosuccinate de dioctylsodium.

3. Procédé selon la revendication 2, dans lequel l'étape de mise en contact consiste à :
a) dissoudre le médicament dans un solvant ;
b) ajouter le médicament solubilisé à une solution comportant au moins un copolymère de vinylpyrrolidone et d'acétate de vinyle pour former une solution claire ; et
c) faire précipiter le médicament solubilisé comportant un copolymère de vinylpyrrolidone et d'acétate de vinyle en tant que stabilisateur de surface en utilisant un agent non solvant.

4. Composition selon la revendication 1 ou procédé selon la revendication 2 ou 3, dans laquelle ou dans lequel le copolymère de vinylpyrrolidone et d'acétate de vinyle contient de 50% à 99,9% de vinylpyrrolidone et de 0,1% à 50% d'acétate de vinyle.

5. Composition selon la revendication 1 ou 4 ou procédé selon l'une quelconque des revendications 2 à 4, dans laquelle ou dans lequel le copolymère de vinylpyrrolidone et d'acétate de vinyle contient 60% de vinylpyrrolidone et 40% d'acétate de vinyle.

6. Composition selon l'une quelconque des revendications 1, 4 et 5 ou procédé selon l'une quelconque des revendications 2 à 5, dans laquelle ou dans lequel le copolymère de vinylpyrrolidone et d'acétate de vinyle contient 80% de vinylpyrrolidone et 20% d'acétate de vinyle.

7. Composition selon l'une quelconque des revendications 1 et 4 à 6, ou procédé selon l'une quelconque des revendications 2 à 6, dans laquelle ou dans lequel le médicament est présent dans une quantité sélectionnée allant de 99,9% à 0,1%, de 80% à 5,0% et de 50% à 10%, en poids sur la base du poids combiné total de la substance médicamenteuse et du stabilisateur de surface, en n'incluant aucun autre excipient.

8. Composition selon l'une quelconque des revendications 1 et 4 à 7, ou procédé selon l'une quelconque des revendications 2 à 7, dans laquelle ou dans lequel au moins un copolymère de vinylpyrrolidone et d'acétate de vinyle est présent dans une quantité sélectionnée allant de 0,1% à 90%, de 1 à 75%, de 10 à 60% et de 10 à 55% en poids sur la base du poids combiné total de la substance médicamenteuse et du stabilisateur de surface en n'incluant aucun autre excipient.

9. Composition selon l'une quelconque des revendications 1 et 4 à 8 ou procédé selon l'une quelconque des revendications 2 à 8, dans laquelle ou dans lequel le médicament est sélectionné à partir du groupe constitué d'un médicament en phase cristalline et d'un médicament en phase amorphe.

10. Composition selon l'une quelconque des revendications 1 et 4 à 9, ou procédé selon l'une quelconque des revendications 2 à 9, comprenant, de plus, au moins un stabilisateur de surface auxiliaire en plus du copolymère de vinylpyrrolidone et d'acétate de vinyle.

11. Composition selon la revendication 10 lorsqu'elle dépend de l'une quelconque des revendications 1 et 4 à 9, ou procédé selon la revendication 10 lorsqu'il dépend de l'une quelconque des revendications 2 à 9, comprenant, de plus, au moins un stabilisateur de surface auxiliaire en plus d'au moins un copolymère de vinylpyrrolidone et d'acétate de vinyle.

12. Composition ou procédé selon la revendication 11, dans lesquels le stabilisateur de surface auxiliaire comprend du sulfate de lauryle.

13. Composition selon l'une quelconque des revendications 1 et 4 à 12, ou procédé selon l'une quelconque des revendications 2 à 12, dans laquelle ou dans lequel la dimension particulaire moyenne efficace de la nanoparticule est inférieure à 1500 nm.

14. Composition selon l'une quelconque des revendication 1 et 4 à 13, ou procédé selon l'une quelconque des revendications 2 à 13, dans laquelle ou dans lequel la dimension moyenne efficace de la nanoparticule est inférieure à 1000 nm.

15. Composition selon l'une quelconque des revendications 1 et 4 à 14, ou procédé selon l'une quelconque des revendications 2 à 14, dans laquelle ou dans lequel la dimension particulaire moyenne efficace de la nanoparticule est inférieure à 800 nm.

16. Composition selon l'une quelconque des revendications 1 et 4 à 15 ou procédé selon l'une quelconque des revendications 2 à 15, dans laquelle ou dans lequel la dimension particulaire moyenne efficace de la nanoparticule est inférieure à 600 nm.

17. Composition selon l'une quelconque des revendications 1 et 4 à 16, ou procédé selon les revendications 2 à 16, dans laquelle ou dans lequel la dimension particulaire moyenne efficace de la nanoparticule est inférieure à 400 nm.

18. Composition selon l'une quelconque des revendications 1 et 4 à 17, ou procédé selon l'une quelconque des revendications 2 à 17, dans laquelle ou dans lequel la dimension particulaire moyenne efficace de la nanoparticule est inférieure à 300 nm.

19. Composition selon l'une quelconque des revendications 1 et 4 à 18 ou procédé selon l'une quelconque des revendications 2 à 18, dans laquelle ou dans lequel la dimension particulaire moyenne efficace de la nanoparticule est inférieure à 200 nm.

20. Composition selon l'une quelconque des revendications 1 et 4 à 19 ou procédé selon l'une quelconque des revendications 2 à 19, dans laquelle ou dans lequel la dimension particulaire moyenne efficace de la nanoparticule est inférieure à 100 nm.

21. Composition selon l'une quelconque des revendications 1 et 4 à 20 ou procédé selon l'une quelconque des revendications 2 à 20, dans laquelle ou dans lequel la dimension particulaire moyenne efficace de la nanoparticule est inférieure à 50 nm.

22. Composition selon la revendication 1 ou selon l'une quelconque des revendications 4 à 21 lorsque dépendante de celle-ci, destinée à être utilisée comme médicament.

23. Composition selon la revendication 21 à utiliser comme un médicament, dans laquelle le médicament est un médicament organique.

24. Composition pharmaceutique comportant une composition nanoparticulaire selon la revendication 1 ou selon l'une quelconque des revendications 4 à 21 et un support pharmaceutiquement acceptable.
